# EUROPEAN PATENT APPLICATION

(11) **EP 3 281 691 A1**
(43) Date of publication of application: **14.02.2018**
(21) Application number: 16776581.7
(22) Date of filing: 06.04.2016
(51) Int. Cl.: B01F 3/04, A61B 8/06, A61K 49/00, B01F 11/00, A61K 9/10, A61K 9/127, A61K 47/02, A61K 47/12, A61K 47/24, A61K 47/26, A61K 47/28, A61K 47/42

(54) **BUBBLE PRODUCTION METHOD**

(30) Priority: 08.04.2015 JP 2015079625
(71) Applicant: Sonocore Inc., Tokyo 100-0005 (JP)
(72) Inventor: TACHIBANA, Katsuro, Fukuoka-shi Fukuoka 814-0180 (JP)
(74) Representative: Herrero & Asociados, S.L.
(86) International application number: PCT/JP2016/061294
(87) International publication number: WO 2016/163415

(57) **Abstract**

A method for manufacturing bubbles of the present invention includes: mixing an aqueous liquid 10 with a gas 3; and applying vibration (an external force) to the aqueous liquid 10 and the gas 3 to generate a plurality of bubbles 1 constituted with the gas 3 in the aqueous liquid 10. A diameter of the bubbles 1 to be generated is adjusted by changing a kind and/or an amount of an additive to be added to the aqueous liquid 10. In this way, it is possible to provide a method for manufacturing bubbles that makes it possible to easily and reliably adjust the diameter of the bubbles to be generated. The additive to be added to the aqueous liquid 10 includes at least one kind of additive among a protein, salts, and sugars.

## Description

### Technical Field

The present invention relates to a method for manufacturing bubbles.

### Background Art

In recent years, in the various fields such as medical care, food, seafood farming, and waste water treatment, the use of micro-sized (about hundreds of micrometers) or nano-sized (about hundreds of nanometers) bubbles has been examined. Generally, the bubbles are constituted with an outer shell, which is constituted with a protein, a lipid, or the like, and a gas sealed in the outer shell.

Particularly, in the medical field, an ultrasonic diagnosis is known which is for making a diagnosis for target site of diagnosis such as chest or abdomen by using such bubbles as an ultrasound contrast agent. During the ultrasonic diagnosis, a diagnosis is made by injecting the bubbles into a blood vessel, irradiating the target site of the diagnosis where the bubbles gather with ultrasonic waves, and making reflected waves (reflection echo) from the target site of the diagnosis into an image.

In recent years, ultrasound therapy using bubbles has been examined (for example, PTL 1). During the ultrasound therapy, bubbles in which a gene or a medical agent (drug) is sealed are injected into a blood vessel and transported to an affected site through the blood flow. When the bubbles reach the vicinity of the affected site, ultrasonic waves are radiated to the bubbles such that the bubbles burst. In this way, the drug sealed in the bubbles can be selectively supplied to the affected site.

As methods for manufacturing the bubbles, a supersaturation bubble generation method and a gas-liquid two-phase flow swirling method are known. The supersaturation bubble generation method is a method in which a gas is dissolved under a high pressure in a mixed liquid containing constituent materials of the bubbles and physiological saline, and then the pressure is reduced such that the bubbles are generated in the mixed liquid. The gas-liquid two-phase flow swirling method is a method in which the aforementioned mixed liquid is stirred at a high speed such that the mixed liquid swirls, a gas is allowed to sufficiently drawn into the swirl, and then the occurrence of swirl is stopped such that the bubbles are generated in the mixed liquid.

However, in the above methods, in order to manufacture bubbles having an intended size, a bubble manufacturing condition needs to be strictly controlled. Furthermore, even if the bubble manufacturing condition is strictly set, the size of the generated bubbles easily changes due to external factors, and it is extremely difficult to control the size of the bubbles.

### Citation List

### Patent Literature

[PTL1] JP-A-2002-209896

### Summary of Invention

### Technical Problem

An object of the present invention is to provide a method for manufacturing bubbles that makes it possible to easily and reliably adjust a diameter (size) of generated bubbles.

### Solution to Problem

The aforementioned object is achieved by the present invention described below in (1) to (10).
(1) A method for manufacturing bubbles, including
   mixing an aqueous liquid with a gas; and
   applying an external force to the aqueous liquid and the gas to generate a plurality of bubbles constituted with the gas in the aqueous liquid,
   wherein a diameter of the bubbles to be generated is adjusted by changing a kind and/or an amount of an additive to be added to the aqueous liquid.
(2) The method for manufacturing bubbles described in (1),
   wherein the additive to be added to the aqueous liquid includes at least one kind of additive among a protein, salts, and sugars.
(3) The method for manufacturing bubbles described in (1) or (2),
   wherein the amount of the additive added to the aqueous liquid is in the range of 0 to 50 w/v%.
(4) The method for manufacturing bubbles described in any one of (1) to (3),
   wherein in a case where the additive is added to the aqueous liquid, a molecule of the additive and/or a component derived from the additive exist in a portion of a gas-liquid interface which is a surface of each of the bubbles and is between the gas and the aqueous liquid.
(5) The method for manufacturing bubbles described in any one of (1) to (4),
   wherein the external force is vibration.
(6) The method for manufacturing bubbles described in (5),
   wherein the aqueous liquid and the gas are added into a container, and
   the container is vibrated such that the aqueous liquid repeatedly collides with an inner surface of the container.
(7) The method for manufacturing bubbles described in (6),
   wherein the container has a long shape and is vibrated to reciprocate in a longitudinal direction of the container and/or rotate mainly in the longitudinal direction.
(8) The method for manufacturing bubbles described in (6) or (7),
   wherein the container has a long shape and is vibrated to reciprocate in a transverse direction of the container and/or rotate mainly in the transverse direction.
(9) The method for manufacturing bubbles described in any one of (6) to (8),
   wherein the container is vibrated in a state where an internal pressure of the container is higher than 1.0 atm.
(10) The method for manufacturing bubbles described in any one of (6) to (9),
   wherein the container is vibrated at a number of revolution of equal to or higher than 5,000 rpm.

### Advantageous Effects of Invention

According to the present invention, by a simple operation of changing a kind (type) and/or an amount of additive added to an aqueous liquid, a diameter of the generated bubbles can be reliably adjusted.

### Brief Description of Drawings

FIG. 1 is a flow chart for illustrating a method for manufacturing bubbles of the present invention.
FIG. 2 shows views (cross-sectional views of a bubble manufacturing container) for illustrating the method for manufacturing bubbles of the present invention.
FIG. 3 is a view for illustrating a state where an aqueous liquid violently collides with an inner surface (top surface) of a container in a step shown in FIG. 2(c).
FIG. 4 is a flow chart for illustrating a second embodiment of the method for manufacturing bubbles of the present invention.
FIG. 5 shows views (cross-sectional views of a bubble manufacturing container) for illustrating the second embodiment of the method for manufacturing bubbles of the present invention.
FIG. 6 is a flow chart for illustrating a third embodiment of the method for manufacturing bubbles of the present invention.
FIG. 7 is a graph showing diameter distributions of bubbles obtained in Examples 1 to 8.
FIG. 8 is a graph showing diameter distributions of bubbles obtained in Examples 8 to 12.

### Description of Embodiments

Hereinafter, a method for manufacturing bubbles of the present invention will be described based on suitable embodiments shown in the attached drawings.

### 1. First embodiment

First, a first embodiment of the method for manufacturing bubbles of the present invention will be described.

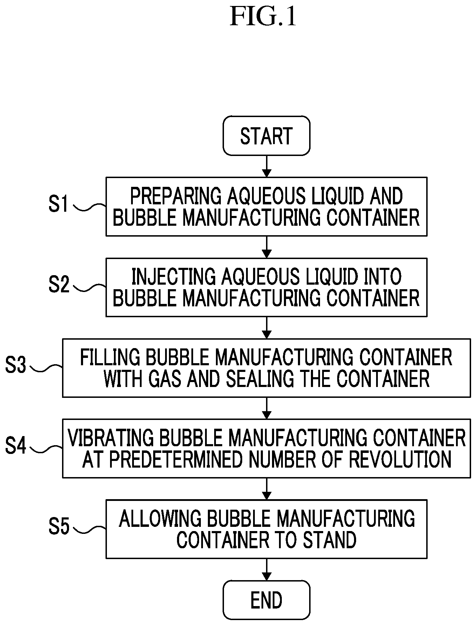

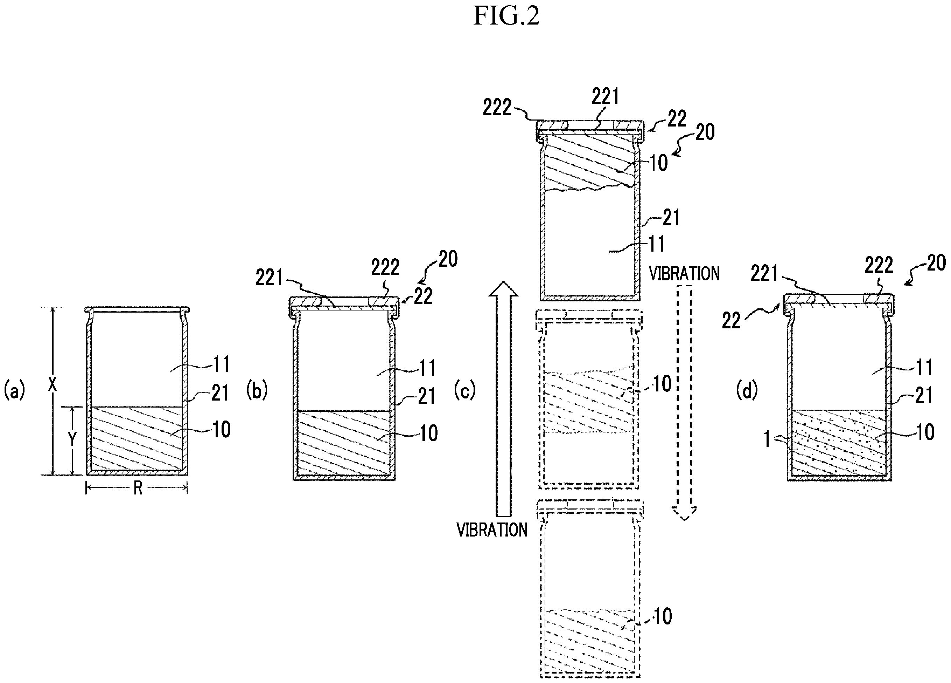

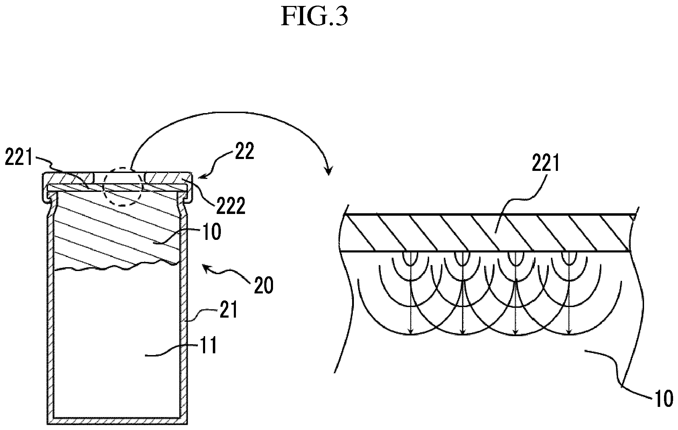

FIG. 1 is a flow chart for illustrating the first embodiment of the method for manufacturing bubbles of the present invention. FIGS. 2(a) to 2(d) are views (cross-sectional views of a bubble manufacturing container) for illustrating the first embodiment of the method for manufacturing bubbles of the present invention. FIG. 3 is a view for illustrating a state where an aqueous liquid violently collides with an inner surface (top surface) of a container in a step shown in FIG. 2(c).

In the following description, the upper side in each of FIGS. 2(a) to 2(d) and in FIG. 3 will be referred to as "top", and the lower side in each of FIGS. 2(a) to 2(d) and in FIG. 3 will be referred to as "bottom".

As shown in FIG. 1, the method for manufacturing bubbles of the present embodiment includes five steps of Steps [S1] to [S5]. Step [S1] is a step of preparing a bubble manufacturing container (hereinafter, simply referred to as "manufacturing container") into which the aqueous liquid is injected. Step [S2] is a step of injecting the aqueous liquid into the manufacturing container to a predetermined height. Step [S3] is a step of sealing the manufacturing container in a state where the manufacturing container is filled with a gas. Step [S4] is a step of vibrating the manufacturing container at a predetermined number of revolution such that the aqueous liquid repeatedly collides with the inner surface of the container. Step [S5] is a step of allowing the manufacturing container to stand.

Hereinafter, these steps will be sequentially described.

### [S1] Preparation step

First an aqueous liquid 10 is prepared. Examples of the aqueous liquid 10 include water such as distilled water, pure water, ultrapure water, deionized water, and RO water. The aqueous liquid 10 may contain a small amount of alcohols.

Then, a manufacturing container 20 (bubble manufacturing container of the present embodiment) is prepared.

The manufacturing container 20 includes a container body 21 accommodating the aqueous liquid 10, and a lid 22 for sealing the container body 21.

The container body 21 is not particularly limited, but preferably has a bottomed cylindrical shape as shown in FIG. 2(a).

In the present embodiment, as the container body 21, a vial having a volume of about 0.5 to 20 ml is used. In the method for manufacturing bubbles, even in a case where such a vial having a small volume is used as the container body 21, when the container body 21 is sealed with the lid 22, because an appropriate pressure is applied to the aqueous liquid 10 in a sealed space within the container body 21, bubbles 1 having a uniform diameter (size) can be stably obtained.

Particularly, in a case where the vial having a volume of about 0.5 to 1.5 ml is used, in a single manufacturing container 20, a liquid containing the bubbles 1 (hereinafter, simply referred to as "bubble-containing liquid") having a volume of about 0.3 to 0.6 ml, which is a volume necessary for a single session of ultrasonic diagnosis, can be manufactured. In this case, at the time of the ultrasonic diagnosis, the bubble-containing liquid in the single manufacturing container 20 can be used up, and hence a waste of the manufactured bubbles (bubble-containing liquid) can be eliminated.

The vial having such a small volume (volume: about 0.5 to 20 ml) has dimensions in which a length X in a longitudinal direction is about 35 to 60 mm and an outer diameter R is about 10 to 40 mm.

As shown in FIGS. 2(b) to 2(d), the lid 22 includes a disk-like rubber stopper (septum) 221 that adheres to a vial mouth of the container body 21 and a fastening portion 222 that fixes the rubber stopper 221 to the vial mouth of the container body 21.

The rubber stopper 221 is not particularly limited, but for example, a rubber stopper made of silicon can be used.

The fastening portion 222 is constituted such that it covers the edge of the rubber stopper 221. On an inner circumferential surface of the fastening portion 222 on the vial mouth side and on an outer circumferential surface of the container body 21 on the vial mouth side, screw grooves that can be screwed with each other are formed (not shown in the drawing). By screwing the screw grooves with each other, the rubber stopper 221 is fixed to the vial mouth of the container body 21 in a state of adhering to the vial mouth. Furthermore, by fastening the fastening portion 222 to the vial mouth of the container body 21, it is possible to fix the fastening portion 222 to the container body 21 in a state where the rubber stopper 221 adheres to the vial mouth of the container body 21.

### [S2] Step of injecting aqueous liquid into manufacturing container

Then, the aqueous liquid 10 is injected into the container body 21 (manufacturing container 20) to a predetermined height. In the present embodiment, as shown in FIG. 2(a), the aqueous liquid 10 is injected to Y [mm]. Accordingly, as shown in FIG. 2(a), the container body 21 into which the aqueous liquid 10 is injected has a void portion 11 on a top portion thereof.

In the present embodiment, in a state where the container body 21 (manufacturing container 20) into which the aqueous liquid 10 is injected is allowed to stand horizontally, provided that a height (length in the longitudinal direction) of the container body 21 is X [mm] and the level of a surface of the aqueous liquid 10 in the container body 21 is Y [mm], it is preferable that a relationship of 0.2 ≤ Y/X ≤ 0.7 is satisfied. In a case where the aforementioned relationship is satisfied, due to the existence of the void portion 11 that is large enough, in Step [S4], it is possible to make the aqueous liquid 10 more violently collide with the top and bottom surfaces and the lateral surface (particularly, the top and bottom surfaces) of the manufacturing container 20. Due to the collision, shock waves occur in the aqueous liquid 10, and hence the bubbles 1 can be easily formed in the aqueous liquid 10.

The aforementioned X and Y more preferably satisfy a relationship of 0.3 ≤ Y/X ≤ 0.5, and even more preferably satisfy a relationship of 0.35 ≤ Y/X ≤ 0.4. In this way, in Step [S4], the bubbles 1 can be more easily formed in the aqueous liquid 10.

### [S3] Step of sealing manufacturing container

Then, the container body 21 is sealed in a state of being filled with the gas 3 (see FIG. 2(b)). Specifically, in the void portion 11 of the container body 21 into which the aqueous liquid 10 is injected, purging is performed using the gas 3, and then the lid 22 is fastened to an opening portion (vial mouth) of the container body 21. In this way, the aqueous liquid 10 and the gas 3 are sealed in the manufacturing container 20.

More specifically, the container body 21 into which the aqueous liquid 10 is injected is moved into a chamber. Thereafter, the internal pressure of the chamber is reduced. Then, the gas 3 is injected into the chamber, and in the atmosphere composed of the gas 3, the lid 22 is fastened to the opening portion of the container body 21. In this way, the aqueous liquid 10 and the gas 3 can be sealed in the manufacturing container 20.

The gas 3 is not particularly limited, and examples thereof include: an inert gas such as air, nitrogen, oxygen, carbon dioxide, hydrogen, helium, argon, xenon, and krypton; sulfur fluoride such as sulfur hexafluoride, disulfur decafluoride, and trifluoromethyl sulfur pentafluoride; low-molecular weight hydrocarbons such as methane, ethane, propane, butane, pentane, cyclopropane, cyclobutane, cyclopentane, ethylene, propylene, propadiene, butene, acetylene, propyne, perfluoropropane, perfluorobutane, and perfluoropentane, or halides thereof; ethers such as dimethyl ether; ketones; esters; and the like. Among these, one kind of substance can be used singly, or two or more kinds of substances can be used in combination.

Among these substances, sulfur hexafluoride, perfluoropropane, perfluorobutane, and perfluoropentane are particularly preferable. The bubbles 1 constituted with these gases 3 exhibit high stability in the body and are more reliably transported to an affected site (target site of treatment) or a target site of diagnosis through blood vessels.

### [S4] Step of vibrating manufacturing container

Then, the manufacturing container 20 is vibrated such that the aqueous liquid 10 repeatedly collides with the top and bottom surfaces and the lateral surface (particularly, the top and bottom surfaces) of the manufacturing container 20. In the present embodiment, as shown in FIG. 2(c), the manufacturing container 20 is vibrated such that the container reciprocates approximately in the longitudinal direction (a vertical direction in FIG. 2(c)) thereof.

In this step, the manufacturing container 20 (lower view in FIG. 2(c)) sealed in Step [S3] is vibrated upwardly (middle view in FIG. 2(c)). As a result, the aqueous liquid 10 moves to the vicinity of the middle of the manufacturing container 20. In a case where the manufacturing container 20 is further vibrated upwardly, the aqueous liquid 10 moves to the top portion of the manufacturing container 20 and collides with the bottom surface (rubber stopper 221) of the lid 22 (upper view in FIG. 2(c)). At this time, as shown in FIG. 3, shock waves occur.

Meanwhile, the manufacturing container 20 (upper view in FIG. 2(c)) is vibrated downwardly (middle view in FIG. 2(c)). As a result, the aqueous liquid 10 moves to the vicinity of the middle of the manufacturing container 20. In a case where the manufacturing container 20 is further vibrated downwardly, the aqueous liquid 10 moves to the bottom portion of the manufacturing container 20 and collides with the bottom surface of the manufacturing container 20 (lower view in FIG. 2(c)). At this time, as shown in FIG. 3, the shock waves also occur.

When the manufacturing container 20 is vibrated in the vertical direction, the aqueous liquid 10 also collides with the inner lateral surface of the manufacturing container 20. At this time, as shown in FIG. 3, the shock waves also occur.

In this way, while being mixed together, the aqueous liquid 10 and the gas 3 are vibrated (applied with an external force), and due to the pressure of the shock waves that occur due to the vibration, the gas 3 is micro-dispersed in the aqueous liquid 10. As a result, a plurality of bubbles 1 constituted with the gas 3 can be generated in the aqueous liquid 10.

By repeating the aforementioned operation, a large amount of bubbles 1 having a uniform diameter can be stably generated in the aqueous liquid 10.

In the method for manufacturing bubbles, in order to obtain the bubbles 1 that are fine enough and have the uniform diameter, the manufacturing container 20 is preferably vibrated at the number of revolution of equal to or higher than 5,000 rpm. As a result, the magnitude (pressure) of the shock waves that occur when the aqueous liquid 10 collides with the manufacturing container 20 becomes high enough, fine bubbles 1 are generated in the aqueous liquid 10, and the diameter of the bubbles can be made uniform. In a case where the number of revolution of the manufacturing container 20 is set to be low within the aforementioned range, the magnitude of the occurring shock waves is reduced, and hence the bubbles 1 having a relatively large diameter can be generated. Furthermore, in a case where the number of revolution is set to be high, the magnitude of the occurring shock waves increases, and hence the bubbles 1 having a relatively small diameter can be generated.

In the present specification, "number of revolution" of the manufacturing container 20 refers to the number of times that the manufacturing container 20 travels the whole vibration route thereof per unit time. For example, in a case where the manufacturing container 20 vibrates at 5,000 rpm, it means that the manufacturing container 20 travels (vibrates) in 5,000 times the whole vibration route for 1 minute.

The number of revolution of the manufacturing container 20 is more preferably equal to or higher than 5,500 rpm, and even more preferably 6,000 to 20,000 rpm. In a case where the number of revolution of the manufacturing container 20 is within the aforementioned range, it is possible to more reliably prevent the bubbles 1 generated by vibration from being destroyed due to collision or from coarsening by being combined with each other. As a result, it is possible to generate a large amount of bubbles 1 having a more uniform diameter in the aqueous liquid 10 with reducing the diameter of the bubbles 1.

As a device that can vibrate the manufacturing container 20 at the number of revolution described above, for example, a bead-type high-speed cell disruption system (homogenizer) can be used. Specifically, Precellys manufactured by bertin Technologies and the like can be used.

The pressure of the shock waves that occur when the aqueous liquid 10 collides with the manufacturing container 20 is preferably 40 kPa to 1 GPa. In a case where the pressure of the shock waves that occur at the time of collision between the aqueous liquid 10 and the manufacturing container 20 is within the aforementioned range, the bubbles 1 generated in the aqueous liquid 10 become finer, and the diameter thereof can be made more uniform. Particularly, the higher the pressure of the shock waves that occur at the time of collision between the aqueous liquid 10 and the manufacturing container 20 is, the finer the generated bubbles 1 can become.

At the time of vibrating the manufacturing container 20, a vibration width of the manufacturing container 20 in the longitudinal direction is preferably about 0.7X to 1.5X [mm], and more preferably about 0.8X to 1X [mm]. In this way, when the manufacturing container 20 vibrates, it is possible to cause the aqueous liquid 10 to reliably collide with the bottom surface of the manufacturing container 20 and the lid 22, and to sufficiently increase the number of times that the aqueous liquid 10 collides with the bottom surface of the manufacturing container 20 and the lid 22. Furthermore, in a case where the manufacturing container 20 is vibrated at the sufficient vibration width described above, the speed at which the aqueous liquid 10 moves in the manufacturing container 20 increases. Therefore, the magnitude of the shock waves that occur when the aqueous liquid 10 collides with the bottom surface of the manufacturing container 20 and the lid 22 sufficiently increases. As a result, it is possible to generate a large amount of fine bubbles 1 in the aqueous liquid 10.

When the manufacturing container 20 is reciprocated in the vertical direction, the manufacturing container 20 is preferably vibrated in a transverse direction (horizontal direction) thereof as well. In this way, the aqueous liquid 10 also collides with the inner lateral surface of the manufacturing container 20, and hence more shock waves can be generated in the aqueous liquid 10. The vibration width of the manufacturing container 20 in the transverse direction is preferably about 0.3X to 0.8X [mm], and more preferably about 0.5X to 0.7X [mm]. In this way, the aforementioned effects are more markedly exhibited.

In this step, it is preferable to vibrate the manufacturing container 20 such that an instantaneous relative speed between the manufacturing container 20 and the aqueous liquid 10 in the manufacturing container 20 becomes equal to or higher than 40 km/h when the aqueous liquid 10 collides with the top and bottom surfaces and the lateral surface of the manufacturing container 20. It is more preferable to vibrate the manufacturing container 20 such that the instantaneous relative speed becomes equal to or higher than 50 km/h. In a case where the aforementioned condition is satisfied, it is possible to sufficiently increase the pressure of the shock waves that occur when the aqueous liquid 10 collides with the manufacturing container 20. As a result, the bubbles 1 generated in the aqueous liquid 10 become finer, and the diameter thereof can be made more uniform.

The period of time for which the manufacturing container 20 is vibrated under the aforementioned condition is preferably about 10 to 120 seconds, and more preferably about 30 to 60 seconds. In a case where the vibration time of the manufacturing container 20 is within the aforementioned range, the number of times that the aqueous liquid 10 collides with the manufacturing container 20 sufficiently increases, and hence a large amount of bubbles 1 can be generated in the aqueous liquid 10. In a case where the vibration time of the manufacturing container 20 is set to be long within the aforementioned range, the amount of bubbles 1 generated in the aqueous liquid 10 can be further increased.

The diameter of the bubbles 1 generated in the aqueous liquid 10 can be adjusted by changing a kind and/or an amount of an additive added to the aqueous liquid 10 as will be described later and by changing the number of revolution of the manufacturing container 20 to be within the aforementioned range. In the present embodiment, by using water as the aqueous liquid 10 as described above, the bubbles having the diameter of about 10 to 1,000 nm can be stably generated.

In the present embodiment, the manufacturing container 20 is vibrated such that the container reciprocates practically in the longitudinal direction thereof, but the method for vibrating the manufacturing container 20 is not limited thereto. For example, the manufacturing container 20 may be vibrated such that the container rotates mainly in the transverse direction and/or the longitudinal direction thereof. Even in this case, the aqueous liquid 10 in the manufacturing container 20 repeatedly collides with the top and bottom surfaces and the lateral surface of the manufacturing container 20, and as a result, the shock waves occur. By using the aforementioned vibrating method, a large amount of bubbles 1 having a uniform diameter can also be stably generated in the aqueous liquid 10.

### [S5] Step of allowing manufacturing container to stand

After the manufacturing container 20 is vibrated under the aforementioned conditions, the manufacturing container 20 is allowed to stand (see FIG. 2(d)). In this way, the large amount of bubbles 1 having the uniform diameter can be stably manufactured in the manufacturing container 20.

It is preferable to perform the aforementioned Step [S2], Step [S3], and Step [S4] by making the temperature of the aqueous liquid 10 remain constant. In this way, the characteristics (viscosity and the like) of the aqueous liquid 10 are stabilized in the manufacturing process of the bubbles, and hence the bubbles 1 having the uniform diameter can be stably generated in the aqueous liquid 10. Examples of the method for making the temperature of the aqueous liquid 10 remain constant include a method of performing each of the aforementioned Steps [S2] to [S4] in a glove box or a thermostatic bath.

Particularly, in the present embodiment, the manufacturing container 20 is vibrated at a high speed in Step [S4]. Therefore, due to the collision between the aqueous liquid 10 and the inner surface of the manufacturing container 20, the manufacturing container 20 easily heated. However, by vibrating the manufacturing container 20 in the thermostatic bath, it is possible to reliably prevent the temperature increase of the aqueous liquid 10. As a result, the bubbles 1 having the uniform diameter can be stably generated in the aqueous liquid 10.

Through Steps [S1] to [S5] described above, the bubbles 1 having the diameter of about 10 to 1,000 nm are manufactured. It is considered that due to a cluster structure of water molecules existing on the surface (gas-liquid interface between the gas 3 and the aqueous liquid 10) of the bubbles 1, the bubbles 1 may be stabilized. The cluster structure of water molecules is mainly constituted with the water molecules and contains a small amount of hydrogen ions (H⁺) and hydroxide ions (OH⁻) which occur due to the ionization of some of the water molecules. The bubbles 1 are charged due to the influence of the ions (particularly, hydroxide ions). It is considered that, as a result, the bubbles 1 may be prevented from being combined with each other due to an electric repulsive force, and hence each of the bubbles 1 may be stabilized.

The inventor of the present invention considered that due to the existence of a certain molecule, ion, radical, or the like on the surfaces of the bubbles 1, the hardness of the bubbles 1 may be improved. Based on this idea, the inventor repeated an experiment of adding various additives to the aqueous liquid 10 when manufacturing the bubbles 1. As a result, as the inventor anticipated, the stability (storage stability) of the bubbles 1 tended to be improved. However, unexpectedly, it was revealed that even if the manufacturing condition (for example, an amount of the additives, the number of revolution of the manufacturing container 20, or the like) is the same, bubbles 1 having different diameters (average diameters) are generated according to a kind of the additive. Furthermore, it was also revealed that even if the same additive is used, the bubbles 1 having the different diameters (average diameters) are generated according to the amount of the additive added.

Based on the aforementioned results, the inventor of the present invention completed the method for manufacturing bubbles of the present invention. That is, in the method for manufacturing bubbles of the present invention, by changing the kind and/or amount of the additive added to the aqueous liquid 10, the diameter of the generated bubbles 1 is adjusted.

The additive added to the aqueous liquid 10 preferably includes at least one kind of additive among a protein, salts, and sugars. By using these additives, the bubbles 1 can be more reliably stabilized. The generated bubbles 1 are constituted with the gas 3, and it is considered that either or both of a molecule of the additive and a component (for example, an ion, a radical, and the like) derived from the additive may be adsorbed onto (exist on) a portion of the surfaces (gas-liquid interface) of the bubbles 1. That is, it is considered that the surfaces of the bubbles 1 may be incompletely covered with the molecule of the additive and/or the component derived from the additive.

Examples of the protein include albumin, globulin, and the like. Examples of the salts include NaCl, KCl, Na₂HPO₄, KH₂PO₄, C₃H₅NaO₃, and the like. Examples of the sugars (including polysaccharide) include glucose, sucrose, dextrin, and the like.

As the aqueous liquid 10 to which the salts are added, for example, physiological saline (0.9 w/v% aqueous NaCl solution) and phosphate buffered saline (PBS) can be used. As the aqueous liquid 10 to which the salts and sugars are added, for example, Nos. 1 to 4 infusion solutions (electrolyte solutions) can be used. As the aqueous liquid 10 to which the protein is added, for example, an albumin preparation, a globulin preparation, and the like can be used. These are on the market as preparations. Therefore, by using these as they are, it is possible to simplify the manufacturing process of the bubbles 1 and reduce the manufacturing costs.

According to the method for manufacturing bubbles described above, even if the additive is not added to the aqueous liquid 10, relatively stable bubbles 1 can be manufactured. Therefore, the amount of the additive added to the aqueous liquid 10 is preferably about 0 to 50 w/v%, more preferably about 0.1 to 40 w/v%, even more preferably about 0.5 to 30 w/v%, and particularly preferably about 1 to 20 w/v%. In a case where the amount of the additive added to the aqueous liquid 10 is set to be within the aforementioned range, highly stable bubbles 1 having the more uniform diameter can be manufactured, although manufacturing of such bubbles 1 also depends on the kind of the additive.

According to the method for manufacturing bubbles described above, it is possible to easily manufacture the large amount of bubbles 1 without requiring a large-scale system. Furthermore, because the sealed container (airtight container) is used for manufacturing the bubbles 1, in a case where the manufacturing container is filled with the aqueous liquid 10 and the gas 3 and then the interior of the container is subjected to a sterilization treatment such as γ-ray sterilization, the bubbles 1 can be manufactured in a sterile environment. Accordingly, the bubbles 1 manufactured in this way can be suitably used in the field of food or medical care.

The bubbles 1 obtained as above can stably exist in the aqueous liquid 10. Therefore, the sealed container containing the obtained bubbles 1 can be stored for a long period of time, such as 6 to 24 months, at room temperature. Furthermore, even after the container is stored for such a long period of time, the number of bubbles 1 in the aqueous liquid 10 substantially is not reduced. Therefore, the sealed container does not need to be vibrated again before use. Accordingly, the sealed container containing the bubbles 1 is easily handled in medical facilities and the like. In addition, because the sealed container having a small volume can be used, the unit cost of the sealed container can be reduced.

The obtained bubbles 1 (bubble-containing liquid) are used for making an ultrasonic diagnosis for patients.

Specifically, first, an injection needle of a syringe is pierced into the rubber stopper of the sealed container. Then, the bubble-containing liquid is aspirated into the syringe from the interior of the sealed container. Thereafter, the injection needle is pulled out of the rubber stopper, and a blood vessel (for example, a vein) of a patient is pierced by the injection needle such that the bubble-containing liquid is injected into the blood vessel. In this way, the bubbles 1 are transported to the affected site (target site of diagnosis) through the blood flow.

Then, at the timing when the bubbles 1 reach a target site of diagnosis, the bubbles 1 are irradiated with ultrasonic waves for diagnosis having a frequency and an intensity at which the bubbles 1 may not burst (the ultrasonic waves are radiated to the bubbles 1). Then, the signals (reflection echo) reflected from the target site of diagnosis are received and subjected to data processing, thereby imaging the target site of diagnosis. In this way, an ultrasonic diagnosis can be made.

As a device performing the irradiation of the ultrasonic waves and receiving the reflection waves from the bubbles 1, known ultrasonic probes can be used.

The bubbles 1 can be used in various fields, in addition to being used for the ultrasonic diagnosis. For example, the bubbles 1 exhibit a germicidal effect with respect to water or food and have an effect of keeping the freshness of food. Furthermore, in an aqueous liquid containing the bubbles 1, water, and oil (hydrophobic component), a large amount of oil can be mixed with water. By exploiting such an effect, it is possible to cook with inhibiting the separation of water from oil in food. Accordingly, the bubble-containing liquid containing the bubbles 1 can also be used in the field of food.

As described above, the bubbles 1 have the diameter of about 10 to 1,000 nm. In a case where the bubbles 1 having the diameter within the above range are injected into the blood vessel by the injection, the bubbles are smoothly transported in the blood vessel due to the blood flow. Particularly, the bubbles 1 (nanobubbles) having the diameter of about 200 to 300 nm are highly stable in a blood vessel. Therefore, such bubbles are reliably transported to a target site practically without being destroyed.

Generally, bubbles constituted with a gas have a property of efficiently reflecting the ultrasonic waves from an interface between a liquid and a gas. Therefore, in the bubbles 1 having the diameter within the aforementioned range, an area of the interface between the gas 3 and the aqueous liquid 10 is large enough, and hence the bubbles 1 are effectively used as an ultrasound contrast agent. Particularly, the nanobubbles exhibit sufficiently high stability. Therefore, the nanobubbles can be used as the ultrasound contrast agent and actively used in various fields described above.

At an affected site where a cancer cell exists, neovessels having a diameter smaller than that of a normal blood vessel extend to the cancer cell from the normal blood vessels around the affected site. The nanobubbles can be smoothly transported even in the neovessels and can reach the cancer cell. Then, by irradiating the bubbles 1 with the therapeutic ultrasonic waves having a frequency and intensity at which the bubbles can burst, it is possible to induce death of the cancer cell. That is, the nanobubbles can be suitably used for cancer treatment. Furthermore, some of the nanobubbles can be passed through the wall of the blood vessel and incorporated into the cancer cell.

The bubbles 1 having the diameter of about 600 to 900 nm can be smoothly transported in blood vessels in the brain, and a position thereof can be clearly specified in an ultrasonic image. Therefore, the bubbles 1 can be suitably used in brain treatment (for example, endovascular treatment of brain).

The diameter (average diameter) of the bubbles 1 can be measured by observation using, for example, a laser diffraction-scattering method, a nanoparticle tracking analysis method, an electric resistance method, an atomic force microscope (AFM), a laser microscope, and the like. As a device for measuring by AFM, for example, it is possible to use a resonant particle measurement system (trade name: ARCHIMEDES) manufactured by Malvern Instruments Ltd.

In the above description, by performing Steps [S1] to [S5], the large amount of bubbles 1 having the uniform diameter can be stably manufactured in the manufacturing container 20. However, in the method for manufacturing bubbles, for example, Steps [S4] and [S5] may be repeated, and in this way, the bubbles 1 having the more uniform diameter can be stably generated.

### <Second embodiment>

Next, a second embodiment of the method for manufacturing bubbles of the present invention will be described.

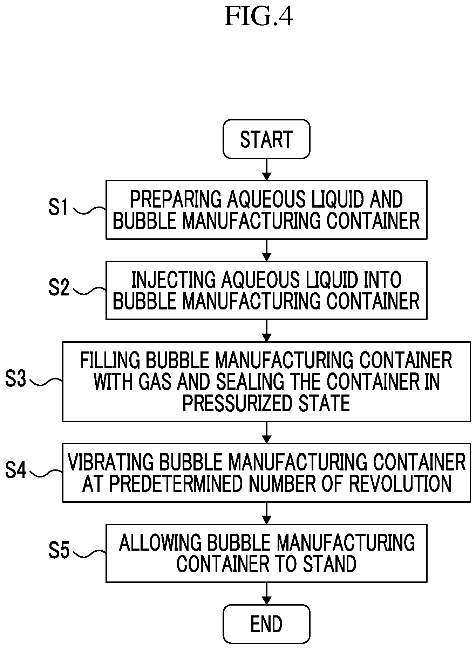

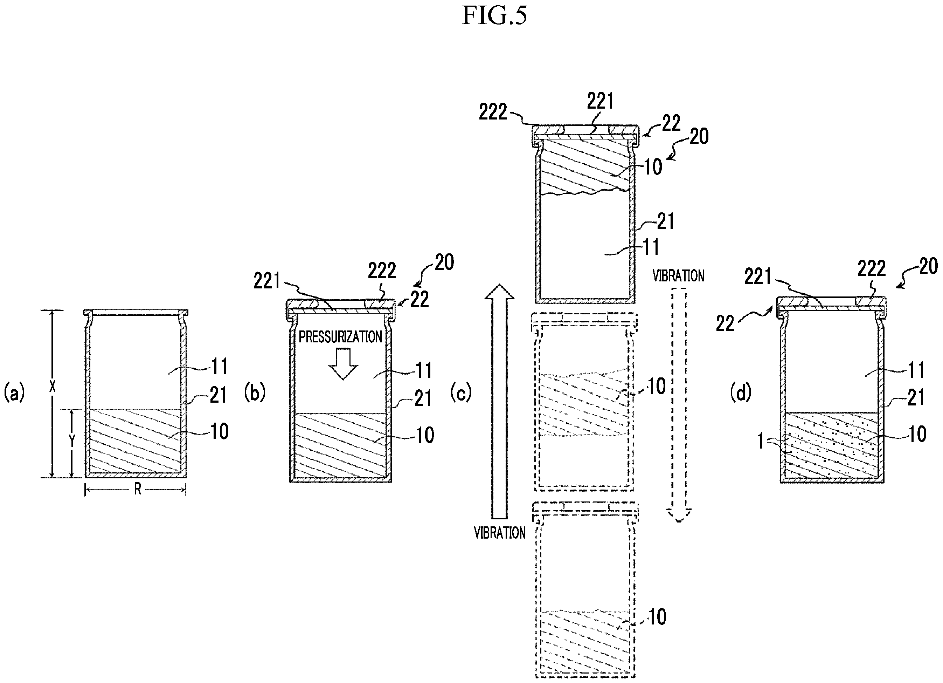

FIG. 4 is a flow chart for illustrating the second embodiment of the method for manufacturing bubbles of the present invention. FIGS. 5(a) to 5(d) are views (cross-sectional views of a bubble manufacturing container) for illustrating the second embodiment of the method for manufacturing bubbles of the present invention.

In the following description, the upper side in each of FIGS. 5(a) to 5(d) will be referred to as "top", and the lower side in each of FIGS. 5(a) to 5(d) will be referred to as "bottom".

Hereinafter, regarding the method for manufacturing bubbles of the second embodiment, differences between the method for manufacturing bubbles of the first embodiment and the method for manufacturing bubbles of the present embodiment will be mainly described, and the same details will not be described.

The method for manufacturing bubbles of the present embodiment is the same as the method for manufacturing bubbles of the first embodiment described above, except that as shown in FIG. 4, in Step [S3] of the first embodiment, the manufacturing container 20 is filled with the gas 3 and sealed in a state where the interior of the manufacturing container 20 is pressurized.

### [S1] Preparation step

First, the aqueous liquid 10 and the manufacturing container 20 of the first embodiment described above are prepared.

### [S2] Step of injecting aqueous liquid into manufacturing container

Then, in the same manner as in the first embodiment described above, the aqueous liquid 10 is injected into the manufacturing container 20.

### [S3] Step of sealing manufacturing container

Thereafter, the container body 21 is filled with the gas 3 and sealed in a state where the interior of the manufacturing container 20 is pressurized (see FIG. 5(b)). Specifically, by using the gas 3, purging is performed in the void portion 11 of the container body 21 into which the aqueous liquid 10 is injected, and then the lid 22 is fastened to the opening portion (vial mouth) of the container body 21. In this way, the aqueous liquid 10 and the gas 3 are sealed in the manufacturing container 20.

Then, a syringe filled with the gas 3 is prepared, and an injection needle of the syringe is pierced into the rubber stopper 221. Thereafter, the gas 3 is further added into the manufacturing container 20 from the syringe, thereby pressurizing the interior of the manufacturing container 20. Subsequently, the injection needle is pulled out of the rubber stopper 221. In this way, it is possible to obtain the manufacturing container 20 which is sealed in a state where the interior thereof is pressurized due to the gas 3.

### [S4] Step of vibrating manufacturing container

Then, in the same manner as in the first embodiment described above, the manufacturing container 20 is vibrated such that the aqueous liquid 10 repeatedly collides with the top and bottom surfaces and the lateral surface of the manufacturing container 20.

### [S5] Step of allowing manufacturing container to stand

Subsequently, in the same manner as in the first embodiment described above, the manufacturing container 20 is allowed to stand.

In the method for manufacturing bubbles of the present embodiment, in Step [S3], the interior of the manufacturing container 20 is caused to be in the state of being pressurized due to the gas 3, and in this way, a portion of the gas 3 is in a state of being micro-dispersed or dissolved in the aqueous liquid 10. Accordingly, in Step [S4], the bubbles 1 easily occur in the aqueous liquid 10, and hence more bubbles 1 having the uniform size can be manufactured than in the method for manufacturing bubbles of the first embodiment described above. Furthermore, the bubbles 1 having a diameter smaller than in the first embodiment can be generated.

The internal pressure (pressure of the gas 3 with which the void portion 11 is filled) of the manufacturing container 20 in Step [S3] is preferably higher than 1.0 atm, more preferably 1.5 to 10 atm, and even more preferably 2 to 5 atm. In this way, a large amount of gas 3 can be micro-dispersed or dissolved in the aqueous liquid 10.

With the method for manufacturing bubbles of the second embodiment, the same operations and effects as in the method for manufacturing bubbles of the first embodiment are also obtained.

### <Third embodiment>

Next, a third embodiment of the method for manufacturing bubbles of the present invention will be described.

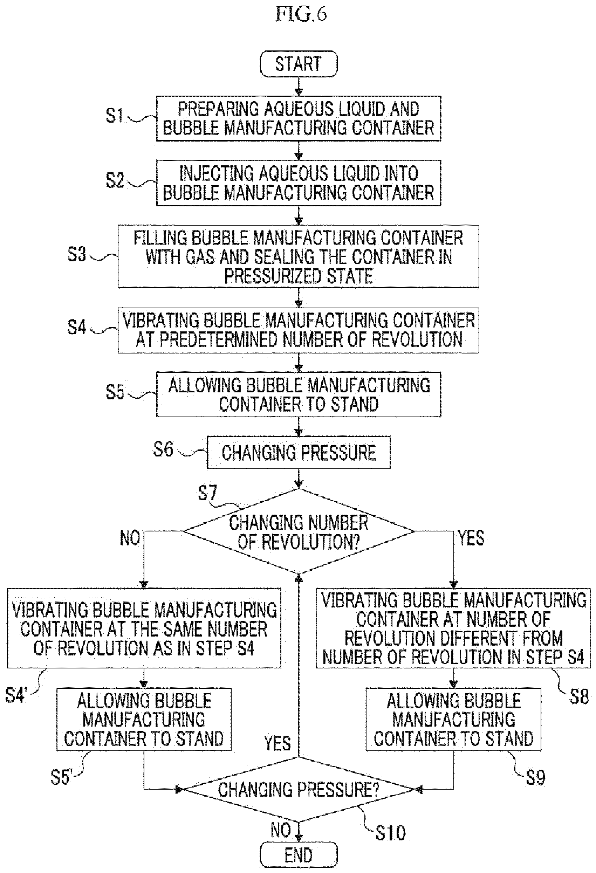

FIG. 6 is a flow chart for illustrating the third embodiment of the method for manufacturing bubbles of the present invention.

Hereinafter, regarding the method for manufacturing bubbles of the third embodiment, the differences between the methods for manufacturing bubbles of the first and second embodiments and the method for manufacturing bubbles of the present embodiment will be mainly described, and the same details will not be described.

As shown in FIG. 6, the method for manufacturing bubbles of the present embodiment includes, after Steps [S1] to [S5] of the first embodiment described above, Step [S6] and Step [S7]. Step [S6] is a step of changing the internal pressure of the manufacturing container. Step [S7] is a step of setting the number of revolution at which the manufacturing container is vibrated.

In a case where the number of revolution is not changed in Step [S7] (in a case where "NO" is selected in Step [S7] in FIG. 6), the method for manufacturing bubbles of the present embodiment further includes Step [S4'] and Step [S5']. In contrast, in a case where the number of revolution is changed in Step [S7] (in a case where "YES" is selected in Step [S7] in FIG. 6), the method for manufacturing bubbles of the present embodiment further includes Step [S8] and Step [S9]. Furthermore, the method for manufacturing bubbles of the present embodiment includes Step [S10] of changing the internal pressure of the manufacturing container. Hereinafter, each of the steps following Step [S6] will be sequentially described.

### [S6] Step of changing internal pressure of manufacturing container

The internal pressure of the manufacturing container 20 having undergone Step [S5] of the second embodiment described above is changed.

### (1) Case where internal pressure of manufacturing container is made higher than pressure in Step [S3]

The interior of the manufacturing container 20 is pressurized in the same manner as in the aforementioned Step [S3]. The gas 3 to be injected may be the same as or different from the gas 3 used in Step [S3]. However, from the viewpoint of the stability of the finally generated bubbles, it is preferable to use the same gas 3.

In such a manufacturing container 20, the internal pressure of the manufacturing container 20 is further increased compared to the internal pressure of the manufacturing container 20 in Step [S3]. Therefore, the amount of the gas 3 which is micro-dispersed or dissolved in the aqueous liquid 10 becomes larger than the amount of the gas 3 which is micro-dispersed or dissolved in the aqueous liquid 10 in Step [S3]. Consequently, when the manufacturing container 20 is vibrated again in Step [S4'] or Step [S8] which will be described later, the bubbles 1 are more easily generated, and as a result, the amount of the generated bubbles 1 increases.

Furthermore, a pressure higher than the pressure applied to the bubbles 1 generated in Step [S4] is applied to the aqueous liquid 10. As a result, the bubbles 1 in the process generation are compressed under a higher pressure, and hence the diameter of the bubbles 1 is easily reduced. Accordingly, it is possible to generate the bubbles 1 having a diameter smaller than that of the bubbles 1 generated in Step [S4].

In this case, the internal pressure of the manufacturing container 20 is higher than the pressure in Step [S3] preferably by 0.5 atm or more, and more preferably by 1 to 10 atm. In this way, it is possible to more reliably generate the bubbles 1 having a diameter smaller than that of the bubbles 1 generated in Step [S4] described above.

### (2) Case where internal pressure of manufacturing container is made higher than 1.0 atm but lower than pressure in Step [S3]

First, an empty syringe is prepared, and then an injection needle of the syringe is pierced into the rubber stopper 221. Thereafter, the gas 3 in the manufacturing container 20 is aspirated into the syringe. In this way, the internal pressure of the manufacturing container 20 is reduced. Then, the injection needle is pulled out of the rubber stopper 221.

In the manufacturing container 20, when the manufacturing container 20 is vibrated again in Step [S4'] or Step [S8] which will be described later, the pressure applied to the aqueous liquid 10 is lower than the pressure applied to the aqueous liquid 10 generated in Step [S4]. As a result, the bubbles 1 in the process of generation are compressed less, and hence the size of the bubbles 1 easily increases. Therefore, it is possible to generate the bubbles 1 having a diameter larger than that of the bubbles 1 generated in Step [S4].

In this case, the internal pressure of the manufacturing container 20 is appropriately adjusted within such a range that the internal pressure becomes higher than 1.0 atm but is lower than the pressure in Step [S3].

### [S7] Step of setting number of revolution at which manufacturing container is vibrated.

After the internal pressure of the manufacturing container 20 is changed as described above, the number of revolution at which the manufacturing container is vibrated again is set.

As in Step [S4] described above, the number of revolution at the time of vibrating again the container is set to be equal to or higher than 5,000 rpm.

In Step [S7], in a case where the number of revolution at the time of vibrating again the manufacturing container 20 is not changed from the number of revolution in Step [S4], that is, in a case where "NO" is selected in Step [S7] in FIG. 6, the following Step [S4'] is performed.

On the other hand, in Step [S7], in a case where the number of revolution at the time of vibrating again the manufacturing container 20 is changed from the number of revolution in Step [S4], that is, in a case where "YES" is selected in Step [S7] in FIG. 6, the following Step [S8] is performed.

### [S4'] Step of vibrating again manufacturing container

In the manner described above, the manufacturing container 20 whose internal pressure is changed is vibrated again at the same number of revolution as in Step [S4] described above. In this way, the bubbles 1 having a diameter different from that of the bubbles 1 generated in Step [S4] are generated in the aqueous liquid 10.

In this step, the manufacturing container 20 is vibrated again at the same number of revolution as in Step [S4]. Therefore, the diameter of the bubbles 1 newly generated in the present embodiment changes according to the pressure changed in Step [S6]. That is, by changing the pressure, the diameter of the bubbles 1 can be adjusted. Consequently, according to the change of the kind and/or amount of the additive added to the aqueous liquid, the bubbles 1 having different intended diameters (average diameters) can be manufactured with excellent reproducibility. Furthermore, because the setting of a device vibrating the manufacturing container 20 does not need to be changed, the bubbles 1 having the different diameters can be manufactured in a simpler manner.

### [S5'] Step of allowing manufacturing container to stand

After the manufacturing container 20 is vibrated under the aforementioned condition, the manufacturing container 20 is allowed to stand in the same manner as in Step [S5]. In this way, the large amount of bubbles 1 having the different sizes can be stably manufactured in the manufacturing container 20.

After the container is allowed to stand, Step [S10] is performed.

### [S8] Step of vibrating again manufacturing container

The manufacturing container 20 whose internal pressure is changed is vibrated again at the number of revolution different from that in Step [S4] described above. In this way, the bubbles 1 having a diameter different from that of the bubbles 1 generated in Step [S4] are generated in the aqueous liquid 10.

### (1) Case where manufacturing container is vibrated again at number of revolution higher than that in Step [S4]

The manufacturing container 20 is vibrated again in the same manner as in Step [S4] described above, except that the number of revolution at which the manufacturing container 20 is vibrated is made higher than the number of revolution in Step [S4].

In this case, the number of revolution of the manufacturing container 20 is not particularly limited as long as it is higher than the number of revolution in Step [S4]. The number of revolution in this case is preferably 6,000 to 20,000 rpm, and more preferably 7,000 to 20,000 rpm. In this way, because the manufacturing container 20 is vibrated at the number of revolution higher than that in Step [S4], it is possible to generate the bubbles 1 having a diameter smaller than that of the bubbles 1 generated in Step [S4]. Furthermore, by setting the number of revolution of the manufacturing container 20 within the aforementioned range, it is possible to more reliably prevent the bubbles 1 generated in Step [S4] and the present step from being destroyed due to collision or from coarsening by being combined with each other. As a result, it is possible to manufacture the bubbles 1 generated in Step [S4] and the bubbles 1 having a diameter smaller than that of the bubbles 1 generated in Step [S4].

### (2) Case where manufacturing container is vibrated again at number of revolution lower than that in Step [S4]

The manufacturing container 20 is vibrated again in the same manner as in Step [S4], except that the number of revolution at which the manufacturing container 20 is vibrated is made lower than that in Step [S4].

In this case, the number of revolution of the manufacturing container 20 is not particularly limited as long as it is lower than the number of revolution in Step [S4]. The number of revolution in this case is preferably 5,000 to 9,000 rpm, and more preferably 5,500 to 7,500 rpm. In this way, the manufacturing container 20 is vibrated at the number of revolution lower than that in Step [S4], and hence the bubbles 1 having a diameter larger than that of the bubbles 1 generated in Step [S4] can be generated. Furthermore, by setting the number of revolution of the manufacturing container 20 to be within the aforementioned range, it is possible to more reliably prevent the bubbles 1 generated in Step [S4] and the present step from being destroyed due to the collision or from coarsening by being combined with each other. As a result, it is possible to manufacture the bubbles 1 generated in Step [S4] and the bubbles 1 having a diameter larger than that of the bubbles 1 generated in Step [S4].

In this step, the manufacturing container 20 is vibrated again at the number of revolution different from that in Step [S4]. Therefore, the diameter of the bubbles 1 newly generated in the present embodiment changes according to the change of the internal pressure of the manufacturing container 20 and to the number of revolution at the time of vibrating again the container. In this way, by changing both the internal pressure of the manufacturing container 20 and the number of revolution at the time of vibrating again the container, it is possible to generate the bubbles 1 having a diameter greatly different from the diameter obtained in the second embodiment described above. Accordingly, in a case where the bubbles 1 that greatly differ from each other in terms of the average diameter are manufactured, the present step is advantageous.

### [S9] Step of allowing manufacturing container to stand

After the manufacturing container 20 is vibrated in Step [S8], the manufacturing container 20 is allowed to stand in the same manner as in Step [S5]. In this way, the bubbles 1 having the different diameters can be stably manufactured in the manufacturing container 20.

After the container is allowed to stand, Step [S10] is performed.

### [S10] Step of changing again internal pressure of manufacturing container

In a case where the internal pressure of the manufacturing container 20 is not changed, that is, in a case where "NO" is selected in Step [S10] in FIG. 6, the method for manufacturing bubbles of the present embodiment is finished. In this way, two kinds of bubbles 1 (first bubbles and second bubbles) having different average diameters within a range of 10 nm to 1,000 nm are manufactured.

In contrast, in a case where the internal pressure of the manufacturing container 20 is changed, that is, in a case where "YES" is selected in Step [S10] in FIG. 6, Step [S7] is performed. Then, Steps [S4'], [S5'], and [S10] described above or Steps [S8], [S9], and [S10] are repeated. In this way, it is possible to manufacture the bubbles 1 having a plurality of different average diameters within a range of 10 nm to 1,000 nm.

In a case where the pressure is repeatedly changed in Step [S10], it is possible to manufacture the bubbles 1 having the plurality of different average diameters according to the number of times the pressure is changed.

In the manner described so far, the bubble-containing liquid is obtained which contains the bubbles 1 having the different diameters in the aqueous liquid 10. The ease of passing the bubbles 1 in blood vessels and the site to which the bubbles are transported vary with the difference in the size (for example, the smaller the diameter of the bubbles 1 is, the farther the bubbles 1 can be transported toward the end of a capillary). Therefore, the bubble-containing liquid obtained as above can be used in many ways according to the purpose of the ultrasonic diagnosis and/or the ultrasound therapy.

With the method for manufacturing bubbles of the third embodiment, the same operations and effects as in the methods for manufacturing bubbles of the first to second embodiments are also obtained.

### Examples

### 1. Manufacturing of bubbles

### (Example 1)

Bubbles were manufactured in the following manner.

### [Preparation step]

Frist, 12 ml of distilled water (aqueous liquid) was prepared, and a 15 ml vial (height X: 50 mm, outer diameter R: 25 mm) was prepared. The vial had the same shape as that of the manufacturing container 20 shown in FIG. 2.

### [Step of injecting distilled water into container]

The distilled water was injected into the prepared vial. A height Y of a surface of the distilled water was 25 mm.

### [Step of sealing container]

Then, by using perfluoropropane (gas), purging was performed in a void in the vial into which the distilled water was injected, and then a lid having the same shape as that of the lid 22 shown in FIG. 2 was inserted into the mouth of the vial. Thereafter, a syringe filled with perfluoropropane was prepared. An injection needle of the syringe was pierced into the rubber stopper of the lid, and 2 ml of perfluoropropane was further added into the vial from the syringe. In this way, a sealed vial having an internal pressure of 2 atm was obtained.

### [Step of vibrating container]

Then, by using Precellys (high-speed cell disruption system) manufactured by bertin Technologies, the sealed vial was vibrated for 30 seconds at the number of revolution of 6,500 rpm. At this time, the sealed vial was caused to reciprocate up and down, and it was confirmed that the distilled water repeatedly collides with the top and bottom surfaces of the sealed vial.

When the sealed vial was vibrated, a vibration width of the sealed vial was set to be 40 mm in a vertical direction and 20 mm in a horizontal direction, such that an instantaneous relative speed between vial and the distilled water became equal to or higher than 40 km/h.

### [Step of allowing container to stand]

After being vibrated, the sealed vial was allowed to stand, thereby obtaining a bubble-containing liquid containing bubbles constituted with perfluoropropane in the distilled water.

### (Example 2)

Bubbles were generated and a bubble-containing liquid was manufactured in the same manner as in Example 1, except that the distilled water was changed to a 1 w/v% aqueous dextrin solution.

### (Example 3)

Bubbles were generated and a bubble-containing liquid was manufactured in the same manner as in Example 1, except that the distilled water was changed to PBS.

### (Example 4)

Bubbles were generated and a bubble-containing liquid was manufactured in the same manner as in Example 1, except that the distilled water was changed to a SOLDEM 3A infusion solution (manufactured by Terumo Corporation).

### (Example 5)

Bubbles were generated and a bubble-containing liquid was manufactured in the same manner as in Example 1, except that the distilled water was changed to a SOLDEM 1 infusion solution (manufactured by Terumo Corporation).

### (Example 6)

Bubbles were generated and a bubble-containing liquid was manufactured in the same manner as in Example 1, except that the distilled water was changed to physiological saline (0.9 w/v% aqueous NaCl solution).

### (Example 7)

Bubbles were generated and a bubble-containing liquid was manufactured in the same manner as in Example 1, except that the distilled water was changed to a 0.25 w/v% aqueous albumin solution.

### (Example 8)

Bubbles were generated and a bubble-containing liquid was manufactured in the same manner as in Example 1, except that the distilled water was changed to a 20 w/v% aqueous glucose solution.

### (Example 9)

Bubbles were generated and a bubble-containing liquid was manufactured in the same manner as in Example 1, except that the distilled water was changed to a 5 w/v% aqueous glucose solution.

### (Example 10)

Bubbles were generated and a bubble-containing liquid was manufactured in the same manner as in Example 1, except that the distilled water was changed to a 10 w/v% aqueous glucose solution.

### (Example 11)

Bubbles were generated and a bubble-containing liquid was manufactured in the same manner as in Example 1, except that the distilled water was changed to a 40 w/v% aqueous glucose solution.

### (Example 12)

Bubbles were generated and a bubble-containing liquid was manufactured in the same manner as in Example 1, except that the distilled water was changed to a 50 w/v% aqueous glucose solution.

### 2. Measurement of diameter distribution of bubbles

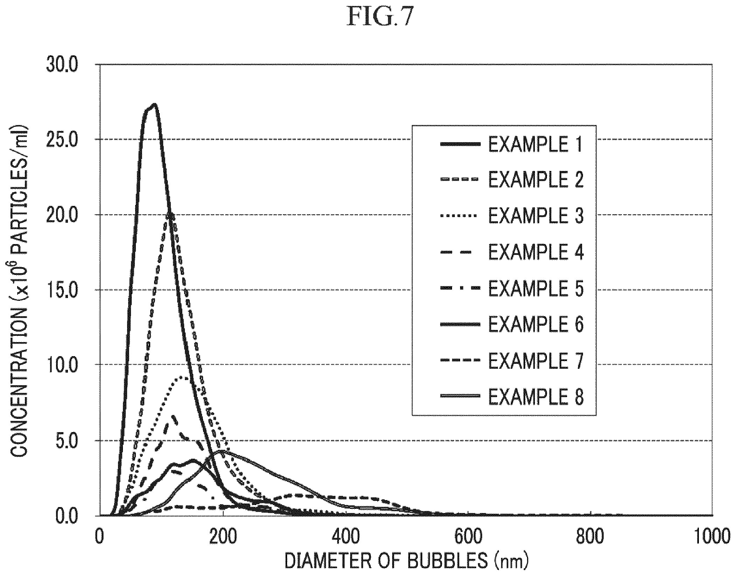

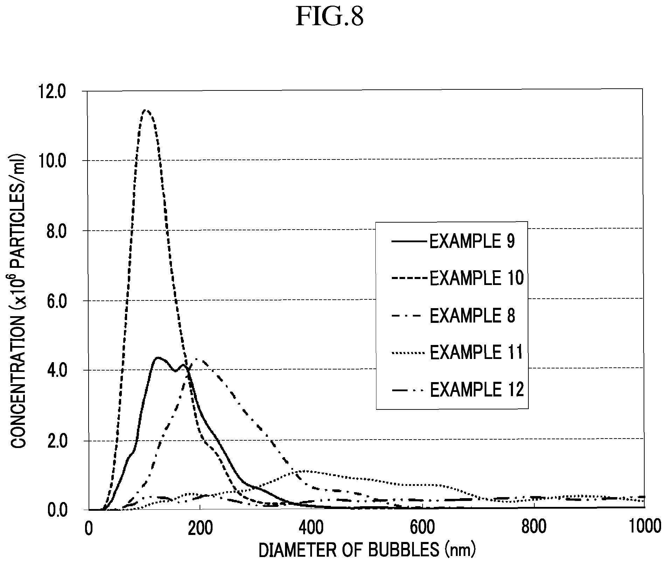

The bubble-containing liquid obtained as above was set in a bubble measurement device (nanoparticle analysis system nanosight), and diameter distributions of the bubbles contained in the bubble-containing liquid (aqueous liquid) was measured. FIGS. 7 and 8 show results.

As shown in FIG. 7, it is understood that depending on whether or not an additive is added to the aqueous liquid, the diameter of the generated bubbles changes. Furthermore, it is understood that the diameter of the generated bubbles also varies with the kind and amount of the additive added to the aqueous liquid.

As shown in FIG. 8, it is understood that in a case where the same additive (glucose) is used, by changing the amount of the additive added to the aqueous liquid, the diameter of the generated bubbles also changes. The inventor of the present invention confirmed that in a case where different additives are added to the aqueous liquid in the same amount, that is, in a case where only the kind of the additive is changed, the diameter of the generated bubbles changes, although this result is not shown in the drawing.

As in the first embodiment, in a case where the interior of the sealed vial is not pressurized in the step of sealing the container, the diameter of the generated bubbles tends to increase. Furthermore, even in a case where the bubbles are manufactured in the same manner as in the third embodiment, according to the kind and/or amount of the additive added to the aqueous liquid, the diameter of the generated bubbles could change.

Hitherto, the method for manufacturing bubbles of the present invention has been described based on the embodiments shown in drawings, but the present invention is not limited thereto.

For example, the method for manufacturing bubbles of the present invention may additionally include one or more steps for a certain purpose.

In Step [S4] of the embodiments described above, by supplying the ultrasonic waves to the aqueous liquid 10 and the gas 3, vibration (external force) may be caused. In this case, the ultrasonic waves preferably have a frequency of about 20 kHz to 10 MHz (particularly, about 200 kHz to 1 MHz) and an intensity of about 10 mW/cm² to 1,000 mW/cm² (particularly, about 100 mW/cm² to 100 W/cm²).

### Industrial Applicability

The method for manufacturing bubbles of the present invention includes a step of mixing an aqueous liquid with a gas with applying an external force to the aqueous liquid and the gas such that a plurality of bubbles constituted with the gas are generated in the aqueous liquid, in which in this step, a diameter of the bubbles generated is adjusted by changing a kind and/or an amount of an additive added to the aqueous liquid. In this way, it is possible to provide a method for manufacturing bubbles that makes it possible to easily and reliably adjust a diameter of the generated bubbles. Accordingly, the present invention is industrially applicable.

### Reference Signs List

1 ··· bubbles, 3 ··· gas, 10 ··· aqueous liquid, 11 ··· void portion, 20 ··· manufacturing container, 21 ··· container body, 22 ··· lid, 221 ··· rubber stopper, 222 ··· fastening portion

## Claims

1. A method for manufacturing bubbles, comprising:
mixing an aqueous liquid with a gas; and
applying an external force to the aqueous liquid and the gas to generate a plurality of bubbles constituted with the gas in the aqueous liquid,
wherein a diameter of the bubbles to be generated is adjusted by changing a kind and/or an amount of an additive to be added to the aqueous liquid.

2. The method for manufacturing bubbles according to claim 1,
wherein the additive to be added to the aqueous liquid includes at least one kind of additive among a protein, salts, and sugars.

3. The method for manufacturing bubbles according to claim 1 or 2,
wherein the amount of the additive added to the aqueous liquid is in the range of 0 to 50 w/v%.

4. The method for manufacturing bubbles according to any one of claims 1 to 3,
wherein in a case where the additive is added to the aqueous liquid, a molecule of the additive and/or a component derived from the additive exist in a portion of a gas-liquid interface which is a surface of each of the bubbles and is between the gas and the aqueous liquid.

5. The method for manufacturing bubbles according to any one of claims 1 to 4,
wherein the external force is vibration.

6. The method for manufacturing bubbles according to claim 5,
wherein the aqueous liquid and the gas are added into a container, and
the container is vibrated such that the aqueous liquid repeatedly collides with an inner surface of the container.

7. The method for manufacturing bubbles according to claim 6,
wherein the container has a long shape and is vibrated to reciprocate in a longitudinal direction of the container and/or rotate mainly in the longitudinal direction.

8. The method for manufacturing bubbles according to claim 6 or 7,
wherein the container has a long shape and is vibrated to reciprocate in a transverse direction of the container and/or rotate mainly in the transverse direction.

9. The method for manufacturing bubbles according to any one of claims 6 to 8,
wherein the container is vibrated in a state where an internal pressure of the container is higher than 1.0 atm.

10. The method for manufacturing bubbles according to any one of claims 6 to 9,
wherein the container is vibrated at a number of revolution of equal to or higher than 5,000 rpm.
